# EUROPEAN PATENT APPLICATION

(11) **EP 4 306 212 A1**
(43) Date of publication of application: **17.01.2024**
(21) Application number: 22184892.2
(22) Date of filing: 14.07.2022
(51) Int. Cl.: B01L 3/00, G06F 16/38, G16H 10/65, A61B 10/00, G01N 1/30

(54) **CONTAINER FOR COLLECTING, TRANSPORTING AND STORING A BIOLOGICAL TISSUE SAMPLE**

(71) Applicant: Milestone S.r.l., 24010 Sorisole (BG) (IT)
(72) Inventor: Visinoni, Francesco, 24040 Mozzo (IT); Minuti, Matteo, 24053 Brignano Gera d'Adda (IT); Martinelli, Michele, 24041 Brembate (IT); Lorenzi, Roberto, 24020 Ranica (IT)
(74) Representative: Kiwit, Benedikt

(57) **Abstract**

The invention refers to a container (100, 300, 400) for collecting, transporting and storing a biological tissue sample of a human or animal, wherein the container (100, 300, 400) comprises a tag (10) with a receiver (12.1) for receiving data relating to the biological tissue sample, a non-volatile memory (11) for storing the data received by the receiver (12.1), and a transmitter (12.2) for transmitting the data stored by the memory (11).

## Description

### Technical field

The present invention relates to a container for collecting, transporting and storing a biological tissue sample, to a system comprising such a container, and to a method using such a container.

### Background art

In the fields of histology, pathology and cell biology, fixation is a critical step in the preparation of histological sections by which biological tissues are preserved from decay, thereby preventing autolysis or putrefaction. The structure of a tissue is determined by the shapes and sizes of macromolecules in and around cells. The principal macromolecules inside a cell are proteins and nucleic acids. Fixation terminates any ongoing biochemical reactions, and it may also increase the mechanical strength or stability of the treated tissues. The broad objective of tissue fixation is to preserve cells and tissue components and to do this in such a way as to allow for the preparation also of thin, stained sections.

For a histological examination of a human or animal tissue sample (specimen), the samples are excided from the patients, such as from an organ of the respective patient, by a clinician in an ambulatory environment or by surgeons during a surgery and then placed in containers. Standard practices call for using containers of different shapes and volume (e.g., buckets, bags, vials) filled with a fixative, in most cases the fixative is neutral buffered formalin 10%, in which the sample is placed.

In particular, there is a need for an exact control of the fixation duration (e.g., the determination of the start of fixation) and for a clear identification of the container and the sample contained.

The container with the sample may then be sent to the histology laboratory or anatomical pathology laboratory for proper analysis. The laboratory can be near to the excision site as well as in a distant place, such as another city or another region.

After the histology laboratory has received the sample, the first operations performed is the accessioning. The accessioning consists of a registration of the incoming sample and insertion of the data in the Laboratory Information System (LIS). In this phase, a further unique identifier is assigned to the tissue sample. The identifier assigned by the laboratory is usually different than the identifier coming from the excision site, since usually the information systems of the two facilities are not connected nor integrated.

Thus, at this stage, the container has several different identifiers. There is a need to reliably read all different identifiers so as to properly track the chain of custody of the sample and thus to catch the dangerous problem of samples mismatching or samples swap.

Furthermore, there is a need that the container provides not only few information, such as only the patient's name or patient/case identifier, but also other relevant information, such as information from the excision site to the laboratory site, such as the precise time on which the sample is placed in formalin at the excision site. Indeed, many regulatory authorities around the world, such as the CAP (College of American Pathologists) in United States of America, provide guidelines for correct fixation of the sample, i.e. provide specific time limits for fixation. Unfortunately, it is not possible to comply to such guidelines if the time when the sample is firstly placed in a fixative such as formalin is unknown or not transmitted to the laboratory where the remaining fixation time is expected to be completed.

Besides, there is a need for a straightforward connection between the excision sites and the laboratories, which often include different information systems and require several requirements regarding protection of sensitive data and cybersecurity.

There is also a need to ensure that information regarding the sample does not become separated or swapped or lost with respect to the container of the sample.

### Summary

According to a first aspect, a container for collecting, transporting and storing a biological tissue sample of a human or animal is provided. The container comprises a (electronic) tag with a receiver for receiving data relating to the biological tissue sample, a non-volatile memory for storing the data received by the receiver, and a transmitter for transmitting the data stored by the memory.

By using the tag, the container does not require, in particular, one or more (printed) identification labels, which visually present information regarding the biological tissue sample (patient's name, patient's code, other patient related information to clearly identify the container, other sample related information, etc.) in a written form (such as with alpha-numeric characters) or in a machine-readable code (such as with a barcode, datamatrix, QR-code or the like) attached to the container. Instead, the container makes it possible that a plurality of information relating to the biological tissue sample is electronically stored in the non-volatile memory of the electronic tag.

In particular, the container makes it possible that a plurality of (respectively unique) identifiers can be assigned to the biological tissue sample, without one or more labels printed and/or stuck on the container. This effects, in particular, that the container can be conveniently used across different information systems that are not connected to one another and/or not integrated. Thereby, the container can be in particular used when an identifier assigned by a laboratory is different from the identifier coming from the excision site, where the information systems of the two facilities are usually neither connected nor integrated.

By the container comprising the tag configured to store electronically a plurality of sample related information, the container is not dependent on the space or area provided by the outer surface of the container in order to include information about the biological tissue sample. Also, there is no problem that a plurality of labels with different identifiers may overlap with one another, making it impossible to read the background or obstructed labels for a human being as well as for a machine (e.g., barcode readers or the like). Further, by the container, there is no problem of providing labels that are bigger than the container itself or that a label stuck on the container is attached in a disadvantageous manner, such as crumpled or attached starting from the side of the container and then partially folded over the lid or the bottom, which would make it difficult or impossible to read information of the label. Hence, the container effects that the data and thus information provided by the tag can be easily received, or drawn, even when the container must provide data from many different sites (surgeon's operating room, laboratories, etc.). Thus, the container particularly facilitates to properly read many different identifiers and to properly track the chain of custody of the sample, whereby it is in particular possible to catch dangerous problems of the sample, such as a sample mismatching or a sample swap.

In particular, to store data relating to the biological tissue sample, the container does not require much space (e.g., comprised by an outer surface of the container) as it is the case for visual labels, which - due to providing the information in a visual form - can provide only very limited or few information such as usually only the patient's name or patient/case identifier. Accordingly, the container facilitates to receive, store and transmit (transfer) much more information regarding the biological tissue sample than a visual label, such as information from the excision site to the laboratory site, e.g. the precise time on which the sample is placed in a fixative (such as formalin) at the excision site. In particular, the container makes it possible to comply easily with several guidelines (such as the guidelines of the CAP in United States of America), because the container can reliably store, in particular, the time when the sample is firstly placed in the container or in a fixative filled in the container.

Further, the container can effect that information regarding the biological sample (such as the fixation start time) does not need to be stored in a database, such as at the excision site, in which database the information is linked to a sample identification code and stored to be shared through an internet connection between different places, such as between the excision site and the laboratory. Accordingly, the container makes it possible to share information regarding the sample between different facilities (such as excision sites and/or laboratories) independent from a communication network (internet, LAN, WAN, etc.) connecting these facilities with one another. In other words, the container provides an easy sharing of information between the facilities even when there exists no straightforward connection (communication protocol, etc.) between them. The container can effect a very secure sharing of the data of the biological tissue sample stored in the memory, because the so provided ability of the container to receive, store and transmit data relating to the biological tissue sample in particular does not require a compliance with a plurality of standards for protecting sensitive data and for fulfilling requirements as to cybersecurity, as it is the case in transmitting data via a network such as the internet.

By the container's (electronic) tag it is further possible that forms accompanying the sample's container (such as a requisition form) and containing much more information regarding the sample are omitted. Thereby, it is particularly avoided that information provided by such forms, which are usually not firmly united to or with the container, becomes separated or swapped or lost.

The container may be filled with a (first) component being a fixative (such as a fixative reagent). The fixative may be a fluid or a liquid. The fixative may be a solution. According to an embodiment, the fixative is formalin, such as 10% neutral buffered formalin (NBF), zinc-formalin or glutaraldehyde or any other fixative used in histology. The container may be filled with the fixative only or with a mixture comprising the fixative. The mixture may be homogenous or heterogeneous or with multiple phases.

The container may be filled with a further (i.e. second) component being a fluid such as a liquid, wherein the further component has a lower specific gravity than the fixative and is immiscible with the fixative such that the component is stratified on top of the fixative. Thereby, the further component forms a protective film to prevent fixative fumes to escape from the container. The further component may be a liquid, a jelly, a gel, a foam or the like. The further component can be a solution, preferably a single solution or a blended solution of organic or inorganic components. Moreover, the further component maybe less toxic than the fixative and preferably has no or just a low toxicity for human beings by inhalation. According to an embodiment, the further component can be made by mineral oils or a mixture of mineral oils, hydrocarbons or a mixture of hydrocarbons, or by isoparaffin C9-C12. The further component may be in a liquid, jelly or foamy state/form. The further component may contain a dye to better distinguish the second component from the fixative. According to an embodiment, the volume of the further component is from 0.1% to 50% of the total volume of both the (first) component and the further (second) component, more preferably between 3% to 5%.

The further component maybe stratified (i.e. layered) on top of the fixative to form a protective film to prevent fixative fumes to escape from the container. In other words, the component may form a lower layer being provided in the container while the further component may form a top layer being provided on top of the lower layer (i.e. the component) and preferably in plane contact with a top surface of the component in the container. To allow a secure protection of escaping fumes of the fixative, the further component may completely cover the top surface of the first component in the container. Due to its material properties and fluid (i.e. flowable) characteristics, the further component may spread over the top surface of the first component once being applied (i.e. poured into the container and onto the component) to completely cover the upper surface of the component thus forming a corresponding protective film preventing the escape of fixative fumes.

The component and preferably the further component are preferably filled into the container before the biological tissue sample is placed in the (first) component. For particular applications or in case the samples must not get into contact with the further component, the further component can also be filled into the container after the biological tissue sample has been placed into the component being (pre-)filled in the container. The filling of the components into the container as well as the placement of the biological tissue samples into the container can be carried out manually, (partially) semi-automatic or (partially) automatic.

The container is not limited to a specific volume or to a specific shape. The container may comprise a sidewall delimiting a space in which the biological tissue sample may be placed. The sidewall may extend from a bottom that delimits the space. The tag may be attached to the sidewall or, if present, to the bottom. The tag may be attached to an outer surface of the container. The outer surface is directed such that it faces away from the space that contains the biological tissue sample. The container may be, for example, a vial, a bag, a pouch, or a bucket. The container may have a volume of between 5ml and 15,000ml (i.e. 15 liters), preferably between 0.5l and 15l. The container may be a single use container, which means that the tag may not be re-used for another biological tissue sample once the biological tissue sample is removed from the container.

The data relating to the biological tissue sample may include a time stamp. Accordingly, the memory is in particular configured to store data including a time stamp. Thereby, an advantageous tracking of one or more characteristics of the biological tissue sample can be effected. The time stamp may be configured to relate to a fixation of the biological tissue sample contained in the container, in particular to indicate the beginning of fixation of the biological tissue sample contained in the container, i.e. when the sample is firstly put in contact with the fixative in the container. Accordingly, the period of time, during which the biological tissue sample is in a defined state (such as in contact with a fixative), can be easily determined without relying on other means such as a database or a form. In particular, by the time stamp it can be easily ensured that a specific time limit for fixation (e.g. prescribed by a guideline or norm) is not exceeded.

The time stamp may comprises a date, such as in the form MM-YYYY or in the form DD-MM-YYYY, wherein "MM" indicates the respective month (01, 02,...12), "YYYY" indicates the respective year (2022, 2023,...), and "DD" indicates the respective day (e.g., 01, 02,..,30). The time stamp may comprise a fraction of the date, such as an hour (00, 01,..., 24; "hh") and preferably a minute (00, 01,..,60, "mm") and more preferably a second (00, 01,..,60; "ss"). In other words, the time stamp may be in the format DD-MM-YYYY-hh or DD-MM-YYYY-hh:mm or DD-MM-YYYY-hh:mm:ss. The time stamp may be configured to indicate the time approximately relative to the respective (coming or current) hour or minute, in particular in 1h- or 5min-steps. For example, when the current time is 11:23h, the time stamp may be configured to indicate this time approximately by indicating 11h or 12h, or 11:20h or 11:25h. The time stamp may comprise a time zone such as CET. The time stamp may comprise a format of time such as the time format according to ISO 8601.

The time stamp may be configured to indicate the end of fixation of the biological tissue sample contained in the container, such as when the sample is received by a laboratory to analyze the biological tissue sample.

The memory may be configured to store data including (and/or the data relating to the biological tissue sample may include):
- an identifier of the object from which the biological tissue sample is taken, such as a patient identifier and/or an organ identifier, in particular a patient name, patient codes or identifiers, specimen codes or identifiers;
- a dimension, a weight, and/or a parts count (i.e. number of specimens) of the biological tissue sample, and/or a type of the biological tissue sample; and/or
- information regarding the fixative used in the container, in particular the amount of the fixative, a sample/fixative ratio, a production lot, an expiration date, a shelf life, a supplier name, a fixative name, and/or a producer name.

Additionally or alternatively, the memory may be configured to store data including, but not limited to, (and/or the data relating to the biological tissue sample may include) one or more diagnostic questions, information regarding the container (such as type and/or size of the container), an universally unique identifier (UUID), an operator name, a part number, and/or one or more text notes.

The memory may comprise a first memory section for storing first data and a second memory section for storing second data, wherein each of the memory sections is configured to lock the respective data independently from the data of the respective other memory. Accordingly, the data can be very easily written on the memory, in particular such that a transmitting or writing unit at a specific station (e.g. in a laboratory) is not required to select the respective memory section which is dedicated for the respectively transmitted data. By locking the respective data, the respectively locked data is protected against deletion or modification after the lock is activated. In an embodiment, the memory may be configured to lock all the data stored by the memory. Thus, the lock may be activated for the whole memory instead of for each single section or block. Each memory section may be configured to store (i.e. to be written) at different times, e.g. such that data can be added and/or locked at each step in which the container is handled by an operator or by a machine.

The memory may be configured to encrypt the data and/or to protect the data by a password or passcode.

The memory may be configured such that data stored in, or by, the memory can be written in one shot or can also be partially written at different times, e.g. such that partial data can be added and/or locked at each step in which the container is handled by an operator or by a machine such as one or more transmitting/reading units.

The container may comprise a lid member for selectively closing a top opening of the container. The top opening is the part of the container via which the sample is placed into the container. The tag may be attached to the lid member, such as on an outer surface comprised by the lid member and/or on an inner surface comprised by the lid member so that the tag is exposed to the inside of the container but not exposed to an outside of the container.

The tag may comprise, or may be, an RFID (Radio Frequency Identification) tag and/or an NFC (Near Field Communication) tag.

The container may comprise a visual, machine-readable tag such as a barcode, in particular a one- or two-dimensional barcode (barcode, datamatrix, QR-code, etc.). The visual tag is to be understood as non-electronic, i.e. being free from any electric circuitry such as an antenna (or a coil) or a memory. The visual tag can be used in combination with the (electronic) tag in such a way that visually reading the visual tag (by a visual reading unit such as a barcode scanner) triggers another unit (such as a transmitting unit and/or a reading unit) to read data stored by the memory of the container or to write data into the memory, i.e. to send data to be received by the receiver of the tag of the container. Accordingly, the visual tag in particular increases the convenience of using the container. For example, upon the recognition of the presence of the visual tag by a visual reading unit, the time stamp indicating the starting time of fixation of the biological tissue sample may be automatically sent (e.g. by a transmitting unit) and then received by the tag of the container and then stored by the memory of the tag.

According to a second aspect, a system is provided. The system comprises: a container as described above, a transmitting unit for sending data to be received by the receiver of the container and then stored in the memory of the container, and a reading unit for reading data transmitted by the transmitter of the container.

The transmitting unit and the reading unit may be comprised by a specific station (or site), such as an excision station (site) or fixation station, e.g. placed close to the point where the biological tissue sample is put into the container with a fixative reagent, a station arranged on the path from the excision or fixation site to another station (e.g., a laboratory), a checkpoint to track a transport comprising the container, an accessioning station, a grossing station, or a station on the path between the accessioning and grossing stations.

The system may comprise a plurality of transmitting and reading units, e.g. comprised by different stations, respectively, such as an excision (or fixation) station, a station arranged on the path from the excision (or fixation) station to another station, a checkpoint to track a transport comprising the container, an accessioning station, a grossing station, and a station on the path between the accessioning and grossing stations, respectively. Thereby, each of the transmitting and reading units can send respectively read data to/from the tag of the container, whereby in particular the control of the process using the different stations is improved (e.g., missing a (crucial) process step is avoided). In an embodiment, a single station (e.g. the fixation station) comprises a plurality of transmitting and reading units.

The system may comprise a (electronic) control unit functionally connected to the transmitting unit and the reading unit. The control unit may be an RFID/NFC electronic control board. In particular, the control unit is configured to control (or manage) the transmitting unit and reading unit, in particular such that the transmitting and reading units can communicate according to a defined communication or data transmission standard, such as one or more RFID/NFC standards. The transmitting and reading units and the control unit may be integrated in a single unit or piece, such as with a common or same printed circuit board. The transmitting unit and/or the reading unit (such as a controller, in particular an electronic control board, of the transmitting unit and/or the reading unit) on the one hand and the control unit on the other hand may be connected with one another in many different ways, in particular by one or more wires and/or wirelessly, for example by a connection such as Ethernet, Wi-Fi, Bluetooth, USB, SPI, I²C, RS-232 serial communication, RS-485 serial communication, or combinations thereof.

In particular, the control unit is configured to send and receive data from/to the transmitting and receiving unit(s), wherein such data is then transferred to the tag of the container, such as through an antenna or coil controlled by (e.g. an electronic control board of) the transmitting and receiving units.

The control unit may be configured to receive a signal indicating the presence of the container and, upon receiving the signal, to activate the transmitting unit in order to send, by the transmitting unit, data to the container and/or to activate the reading unit to read data stored by the memory. In other words, the control unit may be configured to determine based on an input, such as an input coming from the transmitting unit and/or the reading unit, that the container is in such a distance that data sent by the transmitting unit can be received by the container and/or data stored in the memory of the container can be read by the reading unit.

The control unit may be configured to be connectable to a computing device (e.g. comprising a user interface such as a display) and/or a (computer) network such as a local area network (LAN) and/or any other network such as a wide area network (WAN) or the internet. Thereby, the control unit makes it possible to send data from an object (such as a client), which maybe located in the network, to the tag of the container, and/or to receive data from the tag of the container and send the so received data to an object, e.g. located in the network. The object may be another device, a database system, another computer and/or a server, such as a Laboratory Information System or a Health Information System. In particular, the computing device and/or the object maybe configured to (e.g. may comprise a software that is configured to) visualize the data received from the tag of the container and/or to enter further data (such as manually) to be stored by the tag of the container.

By the control unit, a user may insert the information or data to be stored by the memory of the tag of the container. Additionally or alternatively, the control unit may be configured to retrieve automatically data from an object (a device, database system, other computer or server, such as a Laboratory Information System or Health Information System) and to activate the transmitting unit to write this data on the memory of the container without the need of any action by the user. The data written by the control unit on the memory of the container may be a result of elaboration or calculation based on information provided by the user and/or information automatically retrieved.

The control unit and/or a computing device connected to the control unit or the transmitting and reading units (e.g. located in the network to which the control unit is connected) may be configured to generate data and/or to make one or more calculations based on the data (tissue sample characteristics, ratio (volume, weight, etc.) of sample vs. fixative, beginning or end of fixation, etc.) received from the tag. The so generated data may be the duration of the already completed time of fixation. The generated data may then be sent, via the transmitting unit, to the (electronic) tag of the container.

The reading unit may be configured to generate the signal indicating the presence of the container. For example, the reading unit may use an electromagnetic field for reading data stored in the memory, wherein the signal is generated by the electromagnetic field triggering the tag of the container to transmit data. The reading unit may generate the signal only when a specific code stored in the memory is read, such as a factory set code (e.g. a code indicating the manufacturer of the container). Thereby, it is ensured that the true or right container is used for storing the data rather than a tag of another device or container not intended to store the data.

The system may comprises a presence sensor configured to detect the presence of the container in a defined area and, upon detecting the presence of the container in the defined area, to generate the signal. The presence sensor may be configured to measure a weight and/or to measure a distance and to generate the signal based on the measured weight and/or distance.

Additionally or alternatively, the system may comprise a user interface configured to generate the signal. The user interface may be manually operable, such as by a user's hand. For example, the user interface may be a (single) pushbutton. In an embodiment, the container is placed in a defined area (such as an area, in which the transmitting unit can send data to the tag and/or the reading unit can receive data from the tag), wherein after the placement of the container in the defined area, the user interface is operated. By the user interface configured to generate the signal, the system can be produced in a very simple and cost-effective manner, in particular without any presence sensor configured to detect the present of the container.

The data sent by the transmitting unit and received by the receiver of the container may include a time stamp configured to relate to a fixation of the biological tissue sample contained in the container. The time stamp may be configured to indicate the beginning of fixation of the biological tissue sample contained in the container, i.e. indicate a (approximate) time when the sample is firstly put in contact with the fixative in the container. The time stamp may comprises a date, such as in the form MM-YYYY or in the form DD-MM-YYYY, wherein "MM" indicates the respective month (01, 02,...12), "YYYY" indicates the respective year (2022, 2023,...), "DD" indicates the respective day (e.g., 01, 02,...,30). The time stamp may comprise a fraction of the date, such as an hour (00, 01,..., 24; "hh") and preferably a minute (00, 01,..,60, "mm") and more preferably a second (00, 01,..,60; "ss"). In other words, the time stamp maybe in the format DD-MM-YYYY-hh or DD-MM-YYYY-hh:mm or DD-MM-YYYY-hh:mm:ss. The time stamp may comprise a time zone such as CET. The time stamp may comprise a format of time such as the time format according to ISO 8601.

For example, the transmitting unit may be configured to generate the time stamp based on a time set in the control unit and/or in any other computing device to which the transmitting unit is functionally connected. The time stamp may be based on, or received from, a time or clock comprised by the control unit or any other device to which the transmitting unit or control unit is connected to, such as a computing device (computer, laptop, etc.) or a network (LAN, internet, etc.).

The time stamp may be configured to relate to the end of fixation of the biological tissue sample contained in the container, such as when the sample is received by a laboratory to analyze the biological tissue sample. The time stamp configured to relate to the end of fixation may be sent by a further transmitting unit different from the transmitting unit sending the time stamp configured to indicate the beginning of fixation the biological tissue sample.

The system may comprise a visual reading unit (i.e. an optical reading unit such as a code reader) for reading a visual tag comprised by the container, such as barcode, in particular a one- or two-dimensional barcode, wherein the visual reading unit is optionally configured to generate a signal upon reading the visual tag and to transmit this signal to the control unit, for example as the signal indicating the presence of the container. The visual reading unit maybe connected, e.g. via the control unit, to the transmitting unit in such a way that the data or information received by the visual reading unit from the visual tag is (automatically) transmitted to the transmitting unit that then transmits the data to the (electronic) tag of the container to be stored in the memory of this tag. Thereby, in particular a very convenient way to store automatically printed information provided by the visual tag in the electronic tag of the same container is provided. The visual reading unit may be arranged in many different ways. For example, the visual reading unit may be a handheld device or a fixed device attached relative to, such as mounted or embedded in, the transmitting and receiving units.

The system may comprise a transfer device for transferring or transporting the container, such as a conveyor, in particular a conveyor belt, and/or a robotic arm, wherein, optionally, the transfer device is functionally connected to the control unit. In particular, the transmitting unit and/or the reading unit may be part of, or embedded in, the transfer device. The transfer device may be an automatic transfer device and/or may be at least part of a laboratory automation system.

The system may comprise a base on which the container can be placed, wherein the base comprises at least part of the transmitting unit and/or at least part of the reading unit and/or, if present, at least part of the control unit. In an embodiment, the control unit may be provided externally or arranged outside the base. In such an embodiment, the control unit may be a computing device with a display. The base may be a housing defining a space in which at least part of the transmitting unit and/or at least part of the reading unit, and/or, if present, at least part of the control unit, can be arranged. Thereby, the transmitting unit and the reading unit and, if present, the control unit can form a unit that is, in particular, easily transportable. In an embodiment, all of the transmitting unit, the reading unit and, if present, the control unit are arranged in, or comprised by, the base. In an embodiment, the base has the form of a table. For example, the base comprises a substantially flat surface on which the container can be placed and/or one or more supporting structures (one or more feet, etc.) upon which the base can stand.

The visual reading unit may be arranged in many different ways relative to the base. For example, the visual reading unit may be arranged on a lateral side of the base (such as on the right side or on the left side of the base). If multiple visual reading units are provided, they may be arranged on different sides (such as different lateral sides), respectively.

The system may comprise a wall that extends from the base so as to extend on a lateral side of the container when the container is placed on the base, wherein the wall comprises at least part of the reading unit and/or at least part of the transmitting unit and/or, if present, at least part of the visual reading unit, and/or, if present, at least part of the control unit. Accordingly, the tag and, if present, the visual tag can be easily read without requiring the user to orientate and/or to position the container in a specific manner. The wall may be a vertical extension. The wall may be arranged on a backside of the base and/or such that the base and the wall form an L-form when viewed in a side view. In particular, the wall may contain an antenna and/or a coil of the transmitting unit and/or the reading unit. The part of the units comprised by the wall, such as the antenna and/or the coil, may extend correspondingly to the perimeter of the wall. The wall may delimit a space in which the respective components may be arranged. The wall may comprise an opening, wherein the visual reading unit is arranged to read a visual tag via the opening. The wall may comprise a section, such as a central part, which section is substantially empty or void. This section may include the opening and/or may be adapted such that at least part of the visual reading unit can be arranged therein. In particular, the visual reading unit may be arranged such that the visual reading unit is at least partially arranged in the opening. In another embodiment, the system may not comprise the wall. Thereby, a very compact system is provided. In particular, the base may not comprise any wall extending from a surface of the base, on which surface the container can be placed.

According to a third aspect, a method is provided, comprising the steps of:
a) providing a container as described above, b) transmitting, by a transmitting unit, data relating to a biological tissue sample, c) receiving, by the receiver of the container, the data, and d) storing, by the non-volatile memory of the container, the data received by the receiver.

The method may further comprise the step of transmitting, by the transmitter of the container, the data stored by the memory.

The method may further comprise the step of receiving (or reading), by a reading unit, the data transmitted by the transmitter of the container.

The method may further comprise, preferably before step a), the step of placing the biological tissue sample in the container.

The method may further comprise the step of transporting the container, e.g. with the sample contained in the container, to or at a specific location such as a laboratory or a grossing station.

The method may further comprise the step of storing the data transmitted by the transmitter of the container on a memory, such as a memory of a control unit, and, preferably, making the so stored data available to be acquired by a device or a client, such as a Laboratory Information System.

The method may further comprise the step of receiving (or reading), by a further reading unit, the data transmitted by the transmitter of the container. The further reading unit may arranged at a different station than where the (first) reading unit is arranged. For example, the further reading unit may be arranged at a grossing station. As such, it may be ensured that the right container with right sample is used during grossing.

The method may further comprise the step of transmitting, by a further transmitting unit, data relating to a biological tissue sample, and receiving, by the receiver of the container, the data transmitted by the further transmitting unit, and storing, by the non-volatile memory of the container, this data received by the receiver. The further transmitting unit may arranged at a different station than where the (first) transmitting unit is arranged. For example, the further transmitting unit may be arranged at a grossing station.

The method may further comprise the step of transferring the biological sample to a histological carrier, such as a histological cassette. This step may be performed after the step of receiving by the further reading unit.

The method may further comprise the step of receiving, by the reading unit, a time stamp stored by the memory and comparing the received time stamp with a current time (such as provided by the control unit) and deciding, based on this comparison, whether a process acting on the biological tissue sample is completed (or not). For example, the comparison may result in the output of a period of time, wherein it is decided whether this period of time exceeds a defined (prescribed, etc.) period of time so as to determine whether the process acting on the biological tissue sample is completed. The process may be a fixation. The method may further comprise the step of waiting for the completion of the process, and the step of proceeding to a next step (such as tissue processing) when the process is completed.

The method may further comprise, for example between the step of placing the sample in the container and the step of placing the container (e.g. on the base) to transmit data by the transmitting unit and/or to send data by the transmitter of the container, the step of applying (attaching, adhering, etc.) a visual tag on the container.

The method may further comprise the (e.g. between the step of placing the container on a base and/or of reading data stored by the memory of the container on the one hand and the step of transferring the container at a different station such as the grossing station on the other hand) the step of transmitting, by the transmitting unit or a further transmitting unit, further data relating to the biological tissue sample. The further data may include a further unique identifier, e.g. from a station (such as a laboratory) different to a station that comprises the (first) transmitting unit.

The method may comprise one or more further steps of transmitting (by the transmitting unit and/or by one or more further transmitting units) and/or one or more further steps of receiving (by the receiving unit or by one or more further receiving units), which maybe performed or placed on the path from the excision site to the laboratory and/or inside a station (such as a laboratory) where the container is received. Such further steps may be provided to track the container and/or to read/write information or identifications from/to the container.

The method may further comprise the step of encrypting the data stored by the memory and/or of protecting the data stored by the memory by a password or passcode.

The method may further comprise the step of locking data stored in a first memory section comprised by the memory independently from data stored in a second memory section comprised by the memory. In particular, by locking the data, the so locked data is protected against its deletion or modification, in particular when further data is stored by the memory. Thereby, the further data may be automatically stored by the second memory.

### Description of preferred embodiments

Further advantages and specific features will now be described with respect to the accompanied figures, according to which:
- Fig. 1: is a schematic side view of a container according to an embodiment of the present invention,
- Fig. 2: is a schematic perspective view of a container according to an embodiment of the present invention,
- Fig. 3: is a schematic side view of a container according to an embodiment of the present invention,
- Fig. 4: is a schematic perspective view of a system according to an embodiment of the present invention,
- Fig. 5: is a schematic perspective view of a system according to an embodiment of the present invention,
- Fig. 6: is a schematic perspective view of a system according to an embodiment of the present invention,
- Fig. 7: is a schematic perspective view of a system according to an embodiment of the present invention,
- Fig. 8: is a schematic perspective view of a system according to an embodiment of the present invention,
- Fig. 9: is a schematic perspective view of a system according to an embodiment of the present invention,
- Fig. 10: is a schematic perspective view of a system according to an embodiment of the present invention,
- Fig. 11: is a schematic perspective view of a system according to an embodiment of the present invention, and
- Fig. 12: is a schematic perspective view of a system according to an embodiment of the present invention.

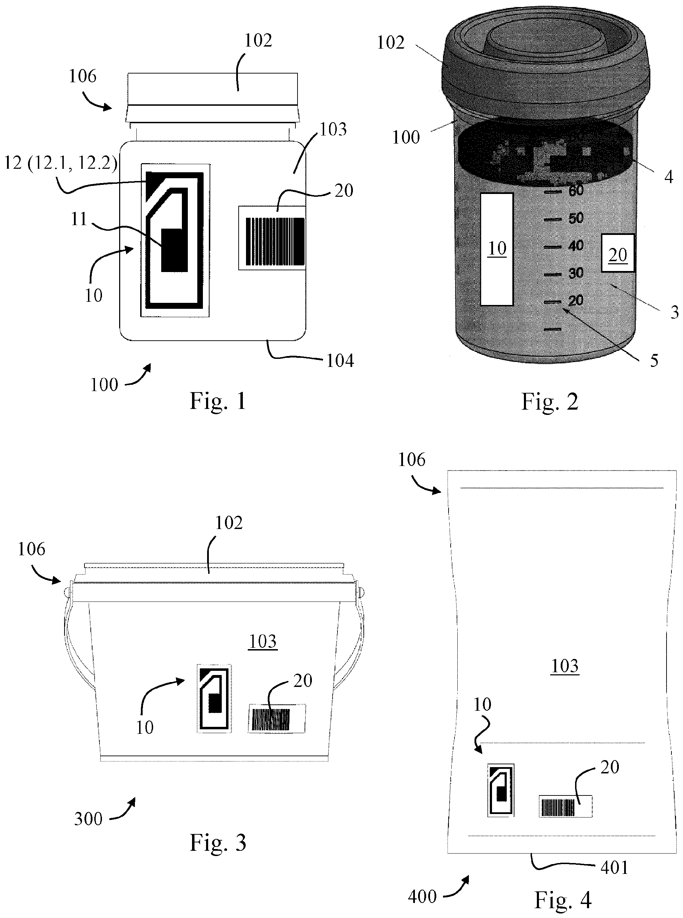

Figures 1-4 show different embodiments of a container for collecting, transporting and storing one or more biological tissue samples according to the present invention. The container may be similar or identical in shape, design, dimensions and composite material to containers commonly used in histology. The container may be made, for instance, of a plastic material but the present invention is not limited to such a material. The container may have a volume of between 5ml and 15l while the present invention is also not limited to this volume which can also be lower or higher according to the particular need and samples (e.g. sample size, type of sample, etc.) to be treated. In an embodiment, the container is made from a transparent material so that the filling level and the position of the biological tissue sample can be identified by the operator from an outside of the container. For example, a (printed) scale (see, for example, the printed scale 5 in the embodiment according to Fig. 2) can be provided on the container to allow for a determination and quantification of the filling level of one or more components to be filled into the container; accordingly, the components may be quantified even without the additional use of a weighing machine. In general, the container is suitable to contain one or more biological tissue samples.

Fig. 1 shows a container 100 according to a first embodiment. The container 100 may have an opening 106 (preferably only one opening 106) which is a top opening and which is used for placing at least a biological tissue sample into the container 100. The container 100 may be filled with a (first) component 3 being a fixative and, optionally, with a further (i.e. second) component 4 being a fluid (such as a liquid) and having a lower specific gravity than the fixative 3 and being immiscible with the fixative 3 such that the component 4 is stratified on top of the fixative 3 in order to form a protective film to prevent fixative fumes to escape from the container 1 (see Fig. 2). The biological tissue sample may be placed (immersed, etc.) in the fixative 3 in order to be fixed. Via the opening 106, the component 3 and, if present, the further component 4 maybe filled into the container 100. Further, via the opening 106, the biological tissue sample may be removed from the container 100. For example, the container 100 may be provided in an empty state to a user, wherein the user then fills the container 100 with the component 3 and, optionally, with the further component 4.

As can be seen in Figs. 1 and 2, the container 100 maybe provided with a lid member 102 for selectively closing and opening the container 100, i.e. selectively closing and opening the top opening 106 of the container 100. For securely fixing the lid member 102 on the container 100, a corresponding fixing means may be provided on the container 100 and/or the lid member 102. The fixing means may be, for instance, a screw joint or bayonet joint provided by a corresponding profiled surface of the matching surfaces of the container 100 on the one hand and the lid member 102 on the other hand. For a tight closure of the container 100, a sealing member (e.g. a sealing gasket) may be provided between the container 100 and the lid member 102 in a closed state of the container 100. The sealing member may be provided on the container 100 (e.g., on a container body) and/or in the lid member 102. It is also possible that the fixing means can be configured by the lid member 102 itself being press-fitted onto the container 100; the press-fitting can be further supported by a corresponding sealing member allowing for a tight press-fit connection.

According to the embodiment shown in Fig. 2, the container 100 maybe (pre-)filled with the one or more components 3, 4. The first component 3 is a fixative, particularly a histological fixative. Usually, the fixative can be formaldehyde which is commonly used in histology as 10% neutral buffered formalin (NBF; that is approx. 3.7% to 4.0% formaldehyde in phosphate buffered saline). The fixative 3 can also be a zinc-formalin or glutaraldehyde or any other suitable fixative for histology. Usually, these fixatives 3 are toxic and are used for preserving the biological tissue sample being placed therein.

The second component 4 is usually filled into the container 100 after the container 1 has been (pre-)filled with the first component 3. The second component 4 is a fluid (e.g. a liquid or liquid solution). The second component 4 has a lower specific gravity (or density or specific weight) compared to the first component 3. Moreover, the second component 4 is immiscible with the first component 3. It is thus possible to stratify the second component 4 on top of the first component 3 so that the second component 4 covers the whole top surface of the first component 3. These two layered components 3, 4 are thus provided in the container 100 such that the first component 3 is at a bottom of the container 1 (preferably opposite the top opening 6) and the second component 4 is placed (preferably in direct and plane contact with the first component 3) on top of the first component 3. Thereby, the second component 4 can form a protective film on top of the first component 3 within the container 100 to prevent fumes of the first component 3 to escape from the container 100. It is thus possible to eliminate or at least severely reduce the fume exposure of the operators involved in the specimens collection and examination.

Just as an example, the first component 3 can be associated to water, e.g. being the NBF basically comprising 4% formaldehyde and 96% water. In this exemplary case, the specific gravity is around 1. In this case, a second component is used having a specific gravity of about between 0,79 to 0,93. However, the present invention is not limited to this example.

The volume of the second component 4 is preferably from 0.1% to 50% of the total volume of both components (i.e. first component 3 + second component 4) and is more preferable between 3% to 5%. It is thus possible to provide a sufficiently thick and thus secure protective film on top of the first component 3.

The second component 4 can be a solution, preferably a single solution or a blended solution of organic or inorganic components. The second component 4 is preferably less toxic than the fixative and more preferable has no or low toxicity for human beings by inhalation. According to a preferred embodiment, the second component 4 can be made by mineral oils or a mixture of mineral oils, hydrocarbons or a mixture of hydrocarbons, or by isoparaffin C9-C12. As an example, the occupational Exposure Limit Values (TLV-TWA) of Isoparaffin hydrocarbons C9-C12 is an average of 300 ppm, while formalin is an average of 0,75 ppm for an eight hour work day. When considering a short-term exposure limit (TLV-STEL), which is the maximum exposure allowed during a 15 minute period, for formalin it is 2 ppm, while for Isoparaffin C9-C12 it is not even reported in the reagent Material Safety Data Sheets (MSDS) as it is irrelevant. The second component 4 can be, for instance, provided in a liquid, jelly or foamy state. According to a preferred embodiment, the second component 4 can contain a dye to better distinguish the second component 4 from the first component 3 when being filled in the container 1 and layered one above the other.

Accordingly, by means of the second component 4, a protective film can be created on top of the first component 3 to prevent its fumes to escape when the container 100 is open; e.g. in case the lid member 2 is opened for placing the sample into or removing the sample out of the container 100. Thereby, toxic fixatives such as formaldehyde can be securely used due to the severe reduction or even elimination of the fume exposure of operators involved in the specimen's collection and examination.

The container 100 may comprise a sidewall 103. The sidewall 103 delimits a space in which the biological tissue sample and, if present, the one or more components 3, 4 can be placed (contained, etc.). The sidewall 103 is not limited to a specific form. For example, the sidewall 103 is curved or has a circular or oval cross-section. The one or more components 3, 4, if present, are in contact with the sidewall 103. The scale 5 may be arranged on the sidewall 103. The sidewall 103 may extend from a bottom 104. When the container 100 is filled with the components 3, 4, only the first component 3 may be in contact with the bottom 104. In other words, the second component 4 may be distanced from the bottom 104.

The container 100 comprises a (electronic) tag (or label) 10 with a non-volatile memory 11, a receiver 12.1 and a transmitter 12.2. In other words, the tag 10 is configured to communicate in order to receive data and in order to transmit (or send) data. The receiver 12.1 and the transmitter 12.2 may be provided separately or together in a single unit, such as in a unit forming a transceiver. By the tag 10, it is thus possible to electronically attach information to the container 100. The tag 10 may comprise an antenna and/or a coil 12 that comprises the receiver 12.1 and the transmitter 12.2. The memory 11 is configured to store (digital) data relating to the biological tissue sample, which is received by the receiver 12.1 or antenna (and/or coil) 12. The memory 11 is a readable and writeable memory, in particular a memory that is writeable more than once. In other words, the memory 11 is a read-write memory. The transmitter 12.2, or the antenna (and/or the coil) 12, is further connected to the memory 11 in such a way that the data stored by the memory 11 can be transmitted, for example to a reading unit.

The memory 11 may comprise one or more electric circuitries. For example, the memory 11 comprises a transponder and/or one or more (micro) chips. The memory 11 may comprise flash memory, such as NOR flash and/or NAND flash. The memory 11 is not limited to a specific memory space. The memory space maybe in a range from 8 bit to 1 gigabit. In an embodiment, the memory space is in a range from 4 byte to 8 Kbyte. The memory 11 may be partitioned in different memory sections or blocks; accordingly, each memory section may contain a portion of the entire memory size. For example, the memory 11 may comprise a first memory section for storing first data and a second memory section for storing second data, wherein each of the memory sections is configured to lock the respective data independently from the data of the respective other memory. Thereby, the respectively locked data is protected against deletion or modification after the lock is activated. The memory 11 may be configured to encrypt the data and/or to protect the data by a password or passcode.

The tag 10 may be provided with or without an own energy source, such as an energy source integrated with the components of the tag 10. In an embodiment, the tag 10 is provided without an own energy source and thus forms a passive tag. This means that the power used for receiving and transmitting data by the tag 10 is drawn - e.g. wirelessly - from an (outside) energy source not comprised by the container or tag 10, such as from a reading and/or transmitting (or writing) unit configured to receive data transmitted by the tag 10 and/or to transmit data to be received by the tag 10. For example, the energy source is configured to transmit radio energy that is used by the tag 10 to be powered in order to receive and transmit data by the receiver 12.1 and by the transmitter 12.2, respectively, such as by the antenna and/or the coil. In an embodiment, the tag 10 may comprise a power input that can be contacted by the energy source so that the energy source supplies the tag 10 with power in order to receive and transmit data. The power input may comprise an electrical contact. In an embodiment, the tag 10 may be provided with, or connected to, an own energy source and thus may form an active tag. The own energy source may be fixed relative to the tag 10, such as integrated in the tag 10 or positioned on a section of the container 100, such as on the sidewall 103 or bottom 104.

In an embodiment, the tag 10 comprises, or is, an RFID tag and/or an NFC tag.

The tag 10 is not limited to a specific position. As shown in Fig. 1, the tag 10 may be provided on the sidewall 103. Alternatively, or additionally, the tag 10 maybe provided on the lid member 102 and/or on the bottom 104 and/or on a top of the container 100. The tag 10 may be arranged on an outer surface of the container 100. Thereby, the tag 10 is not exposed to the inside of the container 100, in particular the biological sample and, if present, the one or more components 3, 4. The tag 10 may be protected by a cover or a housing. The container 100 may comprise a cavity, or a recess, in which the tag 10 is arranged. In particular, the container 100 may comprise an encapsulation that encapsulates and/or isolates the tag 10. In an embodiment, the lid 102 may delimit a space in which the tag 10 is provided. For example, the tag 10 may be attached to an inner surface of the lid 102, which inner surface faces towards the inside of the container 100.

The container 100 may comprise one or more tags 10. In an embodiment, the container 100 may comprise only one tag 10. In another embodiment, the container 100 may comprise a plurality of the tag 10, which may be evenly distributed over the sidewall 103. The tag 10 may extend over more than 50% of the height of the container 100, which height may extend from the bottom 104 to the top of the lid member 102 or any other top of the container 100. Thereby, the tag 10 may be advantageously enlarged in order to provide, for example, more memory space.

The data of the biological tissue sample, which can be received by the receiver 12.1, subsequently stored by the memory 11 and then transmitted by the transmitter 12.2 (e.g. in order to inform a user), can include various information about, or at least related to, the sample. In particular, the data may include a time stamp that is configured to indicate the beginning of fixation of the biological tissue sample contained in the container, i.e. the time when the biological tissue sample is placed into the fixative. For example, in a first step, the biological tissue sample is placed into the fixative filled into the container, wherein in a second step, e.g. (immediately) directly after the first step, the data including the time stamp is sent, e.g. semiautomatically or automatically, to the tag 10 in order to be received by the receiver 12.1 and then stored by the memory 11. In a third step after the second step, the time stamp may be transmitted by the transmitter 12.2, e.g. for being retrieved by a user, such as a user at a location different from the location where the biological tissue sample was fixed. Thereby, the user and/or a computing unit can check how long the biological sample is fixed and if a predefined fixation time has been exceeded. Accordingly, the fixation of the biological sample can be easily controlled and/or verified.

Additionally, or alternatively, data of the biological tissue sample may include: an identifier of the object from which the biological tissue sample is taken, such as a patient identifier and/or an organ identifier; a dimension, a weight, and/or a parts count of the biological tissue sample; and/or information regarding the fixative used in the container.

The container 100 may further comprise a visual tag 20 that is machine-readable, such as by a visual or optical reading unit. The visual tag 20 comprises a code that may be printed. For example, the printed code comprises variously patterned bars. The visual tag 20 may be a barcode, such as a one- or two-dimensional barcode. The code may contain an information regarding the container 100, such as a unique identifier. The code may be configured such that, when read by a visual reading unit, a process is triggered to transmit data to the tag 10 and/or to receive data from the tag 10 and/or to energize the tag 10 so that the tag 10 can receive and/or transmit data. The visual tag 20 may comprise a label with the code printed thereon. The visual tag 20 is not limited to a specific position. For example, the visual tag 20 may be provided on the sidewall 103. The visual tag 20 and the tag 10 may be provided on the same wall of the container 100.

Fig. 3 shows a container 300 according to further embodiment. As shown, the container 300 differs from the embodiment of the container 100 above in particular in that the container 300 has a greater volume. For example, the volume of the container 300 may be in a range from 0.51 to 15l. In particular, the container 300 may be adapted to contain a biological tissue sample in the form of an organ. The container 300 may comprise a handle 301, which may be attached at or near the opening 106 of the container 300. The container 300 may be a bucket.

Fig. 4 shows a container 400 according to a further embodiment. As shown, the container 400 may have the form of a bag or pouch. The container 400 may be formed of one or more sheets that are attached to one another, e.g. by sealing such as heat sealing, to form the space in which the biological tissue sample and, if present, the one or more components 3, 4 may be placed. Thereby, the container 400 may not comprise a lid such as the lid member 106. In particular, the container 400 may comprise a first sidewall 103 and a second sidewall (not shown), which are attached to one another to form or delimit the space. The sidewalls may be connected at one edge or rim of the container 400 in such a way that a bottom 401 is formed, whereby the container 400 can stand in an upright manner.

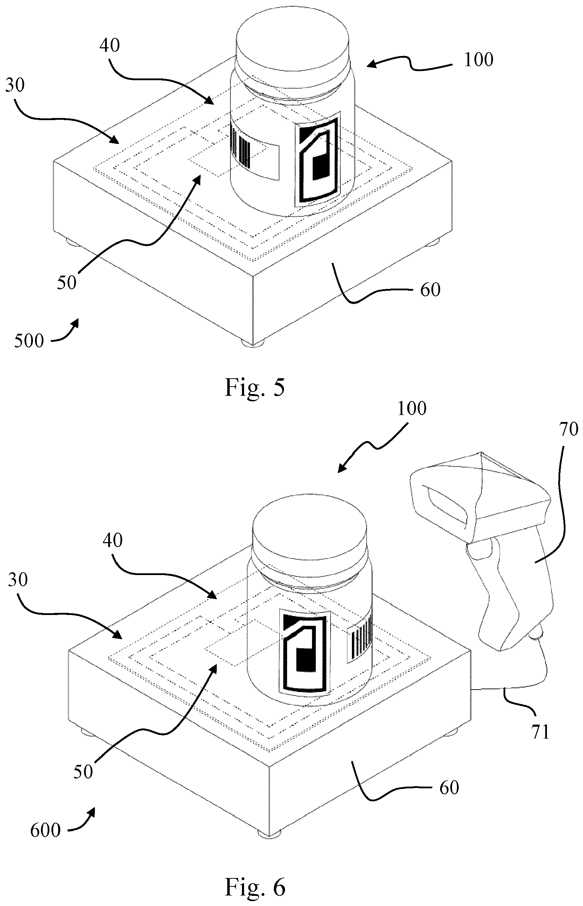

Figures 5-12 show different embodiments of a system of the present invention. These embodiments respectively show a system comprising a container 100. However, each of the systems may also comprise, instead of the container 100, the container 300 or the container 400.

Figure 5 shows a system 500 according to an embodiment. The system 500 comprises one or more transmitting (or writing) units 30 for sending data to be received by the receiver 12.1 of the container 100 and then stored in the memory 11 of the container, and one or more reading (or receiving) units 40 for reading (or receiving) data transmitted by the container. The transmitting and reading units 30, 40 may be integrated in a unit that may comprise an antenna and/or a coil for transmitting and reading data. By the antenna, the data may be transmitted using radio frequency. By the coil, the data may be transmitted based on induction between transmitter and receiver coils, e.g. like a transformer, usually for short distance. The transmitting unit 30 and/or the reading unit 40 may be configured as an energy source connectable, in particular without contact and/or wirelessly, to the tag 10 in order to energize or power the tag 10 so that the tag 10 can receive data (from the transmitting unit 30) and store this data in the memory 11 and/or so that the tag 10 can transmit data stored by the memory to the reading unit 40. For example, one or more of the units 30, 40 may be configured to generate electromagnetic waves (radio waves) and/or electromagnetic induction which power the tag 10; data transmitted to, and received from, the tag 10 is then transmitted by way of these electromagnetic waves and/or electromagnetic induction. In an embodiment, the transmitting unit 30 is an RFID and/or NFC transmitter or writer, and/or the reading unit 40 is an RFID and/or NFC reader.

The system 500 may comprise a control unit 50 that is functionally connected to the transmitting unit 30 and the reading unit 40. Accordingly, the control unit 50 is configured to control the units 30, 40. In particular, the control unit 50 may be configured to receive a signal indicating the presence of the container 100 and, upon receiving the signal, to activate the transmitting unit 30 in order to send, by the transmitting unit 30, data to the container 100 in order to be received by the tag 10 where it is then stored by the memory 11. For example, after insertion of the biological tissue sample in the container 100 (e.g. in order to start a fixation of the biological tissue sample), the signal may be automatically generated, whereby the control unit 50 activates the transmitting unit 30 to send a time stamp (e.g. based on a time set in the control unit 50) to be received by the tag 10 of the container 100. Thereby, the start time of fixation of the biological tissue sample is stored in the memory 11 of the tag 10. If desired, a user can then draw, by the reading unit 40, the time stamp from the memory 11 in order to check or analyze the fixation of the respective biological sample.

The signal indicating the presence of the container 100 may be generated in many different ways. In particular, the reading unit 40 may be configured to generate the signal. For example, the reading unit 40 may be configured to supply energy (such as in the form of electromagnetic waves and/or by electromagnetic induction) to the tag 10 and configured to detect that the tag 10 draws energy or not, e.g. because the tag 10 is out of distance to be supplied by the energy provided by the reading unit 40. Based on this detection, the reading unit 40 can generate the signal and forward the so generated signal to the control unit 100. Additionally or alternatively, the system may comprise a presence sensor configured to detect the presence of the container 100 in a defined area and, upon detecting the presence of the container in the defined area, to generate the signal. Accordingly, the presence sensor may be functionally connected to the control unit 50. The presence sensor may comprise a weight sensor and/or a distance sensor.

Additionally or alternatively, the system may comprise a user interface (not shown) configured to generate the signal. The user interface may be manually operable, such as by a user's hand. Upon activation of the user interface (such as by a user touching and/or pushing the user interface), the signal indicating the presence may be generated, e.g. by simply closing an electric circuit. For example, the user interface may be a (single) pushbutton. The user interface may be part of a base of the system, such as of the base 60 described herein below.

As shown in Fig. 5, the system 500 may comprise a base 60 on which the container 100 can be placed. The base 60 may comprise, or house, the units 30, 40 and, if present, the control unit 50. The units 30, 40 and, if present, the control unit 50 may be completely arranged inside of the base 60 such as in a space delimited by the base 60. The base 60 may comprise a power inlet for supplying the units 30, 40 and, if present, the control unit 50 with electrical power.

Figure 6 shows a system 600 according to a further embodiment. The system 600 comprises in addition to the system 500 a visual reading unit (such as a visual code reader) 70 for reading the visual tag 20. The visual reading unit 70 may be integrated with, or comprised by, the unit 30 and/or the unit 40, such as in a single unit. The visual reading unit 70 may befunctionally connected to the control unit 50. The visual reading unit 70 may beconfigured to generate a signal upon reading the visual tag 20. For example, the visual reading unit 70 is configured such that this signal is only generated when a specific code provided by the visual tag 20 is read, such as a specific (unique) factory set code or a patient code. This signal is then transmitted to the control unit 50. The control unit 50 may receive this signal as the signal indicating the presence of the container 100, whereupon the control unit 50 activates the transmitting unit 30 in order to send data to the tag 10 of the container 100 (such as a time stamp indicating the beginning of fixation of the biological tissue sample) and/or activates the reading unit 40 in order to draw data from the tag 10 of the container 100.

The visual reading unit 70 may be a barcode scanner. As shown in Fig. 6, the visual reading unit 70 may be a handheld device and/or may be connected to the control unit 50 via a wire 71. Alternatively, the visual reading unit 70 may be connected to the control unit wirelessly, i.e. without any wire 71.

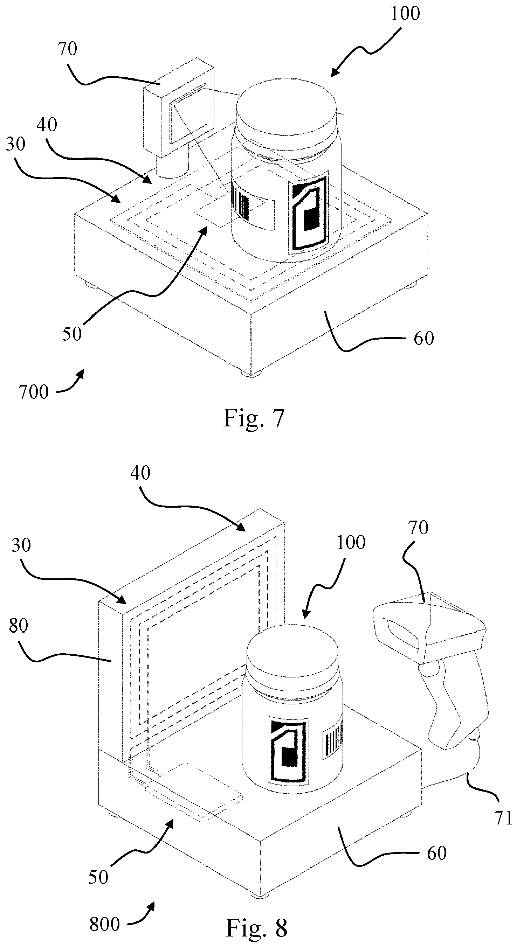

Fig. 7 shows a system 700 according to a further embodiment. In contrast to the system 600, the visual reading unit 70 is attached or fixed to the base 60. The visual reading unit 70 is attached or fixed to the base 60 in such a way that a reading side of the visual reading unit 70 is directed towards the container 100 when placed on the base 60. The visual reading unit 70 may comprise a part that is integrally formed with the base 60. Additionally or alternatively, the visual reading unit 70 may be attached by a form and/or frictional connection to the base 60, such as screwed into the base 60. In particular, the visual reading unit 70 may be attached to the base 60 such that the visual reading unit 70 has a defined position and orientation relative to the surface on which the container 100 can be placed. The visual reading unit 70 may protrude from the surface of the base 60 on which the container 100 can be placed.

Fig. 8 shows a system 800 according to a further embodiment. The system 800 further comprises a wall 80 that extends from the base 60 so as to extend on a lateral side of the container 100 when the container 100 is placed on the base 60. In other words, the wall 80 protrudes from the surface on which the container 100 can be placed. In the system 700, the wall 80 comprises at least part of the transmitting and reading units 30, 40, such as at least part of the one or more antennas and/or coils. In other words, at least part of the transmitting and reading units 30, 40 may be arranged inside of the wall 80 and thus housed by the wall 80. The reading units 30, 40 may be completely arranged inside of the wall 80. The control unit 50 may be arranged inside of the base 60. The wall 80 may extend perpendicular to the base 60, in particular to the surface of the base 60 on which the container 100 can be placed.

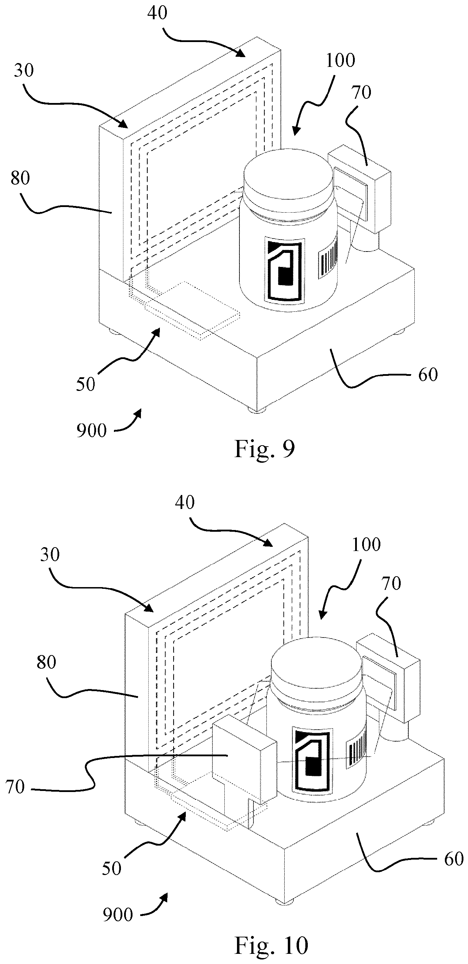

Fig. 9 shows a system 900 according to a further embodiment. In contrast to the system 800, the visual reading unit 70 is not provided as a handheld device but according to the visual reading unit 70 in the system 700. The visual reading unit 70 may be arranged on a side of the base 60 different from the side on which the wall 80 is arranged. For example, the visual reading unit 70 and the wall 80 may be arranged not opposite to one another and/or the visual reading unit 70 may define a plane that extends transversely to a plane defined by the wall 80.

Fig. 10 shows a system 1000 according to a further embodiment. In contrast to the system 900, the system 1000 comprises not a single visual reading unit 70 but a plurality (such as two) visual reading units 70. The further visual reading unit 70 is arranged analogously to the arrangement of the (first) visual reading unit 70 as described for the system 900. In particular, the further visual reading unit 70 may be arranged opposite the visual reading unit 70 so that the container 100 can be placed between these visual reading units. Thereby, the visual tag 20 can be read in a very reliable manner. The visual reading unit 70 may be arranged on a side of the base 60 different from the sides on which the wall 80 and the other visual reading unit 70 is arranged.

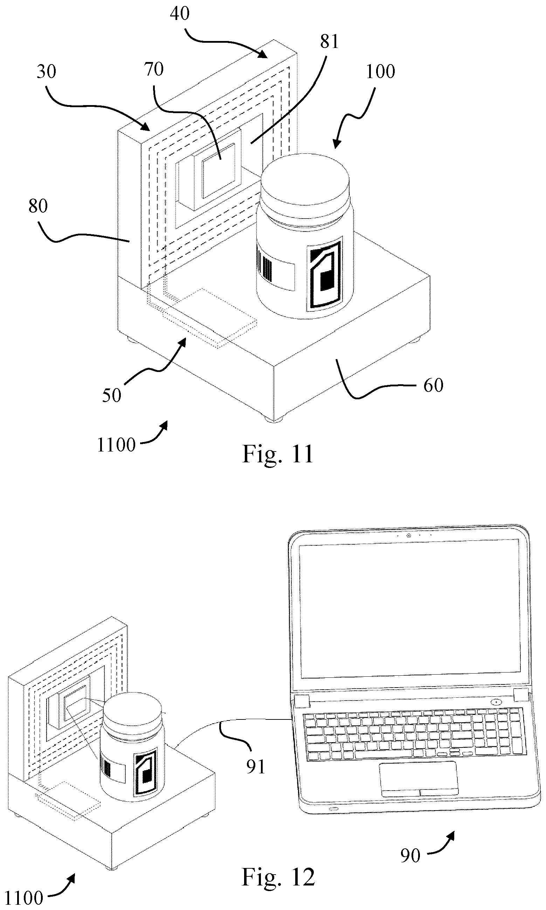

Fig. 11 shows a system 1100 according to further embodiment. In contrast to the system 900, the visual reading unit 70 is not provided separately from the wall 80. Rather, at least part of the visual reading unit 70 is comprised by the wall 80, such as in a recess or a cavity of the wall 80. In particular, the visual reading unit 70 may be at least partially housed by the wall 80. The part of the visual reading unit 70 comprised by the wall 80 may be at least partially, or completely, surrounded by a part of the units 30, 40 (when seen along a direction perpendicular to the wall 80, such as to a surface of the wall facing the container 100, if present), such as by one or more antennas and/or coils of the units 30, 40. The wall 80 may comprise an opening 81, wherein the visual reading unit 70 is arranged to read via the opening 81. Additionally or alternatively, one or more electrical lines of the units 30, 40 may extend at least partially or completely around the part of the visual reading unit comprises by the wall 80.

As shown in Fig. 12, the control unit 50 may be configured to be connectable to a computing unit or device, such as a laptop 90,. The connection to the computing unit may bea wire-bound connection (e.g. via a wire 91) or a wireless connection (nearfield communication such as Bluetooth, WiFi, etc.). For example, the computing unit comprises a user interface via which data relating to the biological tissue sample can be entered, transmitted via said connection to the control unit 50 and, from there, transmitted via the transmitting unit 30 and receiver 12.1 of the tag 10 to the tag 10 of the container 100. Conversely, data relating to the biological tissue sample and stored by the memory 11 of the tag 10 may be transmitted via the transmitter 12.2 of the tag 10 and the reading unit 40 to the control unit 50 and from there transmitted via said connection to the computing unit 90 where the so received data may be presented via the user interface.

Additionally or alternatively, the control unit 50 may be configured to be connectable to a network or computer network, such as a local area network (LAN), a wide area network (WAN) and/or the internet. Accordingly, data drawn from the tag 10 of the container 100 can be sent and/or distributed over a wide range. In particular, the control unit 50 may be configured to be connectable to a system such as a Laboratory Information System (LIS, LIMS, or LMS).

In the following, a preferred method for using the container is described.

In a first step, a biological tissue sample may be placed in a container 100 comprising a tag 10 and being filled with a fixative. This step may be executed at the excision site.

In a second step, which is optional, a visual tag 20 is applied on the container 100. The visual tag 20 may indicate the patient's name or patient's code or other patient related information written as visual code, such as a barcode, a datamatrix, a QR-code or the like. This step is preferably between the before-mentioned first step and the following third step of placing the container 100 in such a way that the tag 10 is read or data is stored by the tag 10.

In a third step, the container 100 is placed close to transmitting and reading units 30, 40, such as on a base 60 comprising at least part of these units. The visual tag 20, if present, may be read manually or automatically by a visual reading unit 70.

In a fourth step, data including a time stamp indicating the beginning or starting of the fixation of the biological tissue sample (e.g., the current date and time) is written on the memory 11 of the tag 10 automatically, e.g. by a control unit 50. If present, even the information of the visual tag 20, read by the visual reading unit 70, may be written on the memory 11. Moreover, if other information is entered using the control unit 50 or any other device connected to the control unit 50, such information is written on the memory 11 as well. Such an automatic writing may be triggered by the presence detection of the tag 10 and/or visual tag 20, e.g. by using the units 30, 40 and/or the unit 70. To operate in this way, the units 30, 40 may continuously monitor for the presence of an electronic tag (such as the tag 10) and/or the unit 70 may continuously monitor for the presence of a visual tag (such as the visual tag 20).

In a fifth step, the container 100 is transported with the biological tissue sample (specimen) to a laboratory.

In a sixth step, the container 100 is placed close to (further) transmitting and reading units 30, 40, such as on a (further) base 60 comprising at least part of these units, of a station such as the laboratory upon reception. Data stored by the tag 10, i.e. memory 11 of the tag 10, is read by the reading unit 40. In particular, the time stamp (such as the date and time) of the beginning of the fixation of the biological tissue sample is read and received. Thus, knowing when the fixation started (such as only the date or specified to the hour or even minute or second), the laboratory is able to calculate the remaining fixation time according to the preferred guidelines. The data read may be stored on the control unit and may bemade available to be acquired by an object, such as a network, in particular a Laboratory Information System.

In a seventh step, which is optional, further data relating to the biological tissue sample, such as a (further) unique identifier assigned by the laboratory, can be sent, by the transmitting unit 30, to the tag 10 and stored by its memory 11.

In an eighth step, the container 100 is transferred to a further station such as a grossing station.

In a ninth step, during the grossing, the right container with the right biological tissue sample is identified by reading the data stored in the tag 10 by a further reading unit 40. Subsequently, the biological tissue sample is transferred to a histological cassette, in which the sample may be placed in a fixative.

In a tenth step, the time stamp indicating the beginning of fixation of the biological tissue sample is compared with the current time. Based on this comparison, the already elapsed fixation time is calculated and compared with the required (proper) fixation time. If the required fixation time has not been reached yet, the biological tissue sample remains in the fixative (i.e. waits) until the proper fixation time of the biological tissue sample is completed. Then, the biological tissue sample proceeds with the tissue processing. During tissue processing, at least a portion and/or a completion of the fixation may occur or may be carried out, e.g., during the first phase of tissue processing.

Moreover, the method may comprise further reading/transmitting (writing) steps using further transmitting and reading units (such as reading/writing stations) placed on the path from the excision site to the laboratory, e.g., checkpoints to track the transport.

Furthermore, the method may comprise further reading/transmitting (writing) steps using further transmitting and reading units placed inside the laboratory to track the container during the transfer or to read or add information or identifications, displaced in several checkpoints along the path between the accessioning and grossing.

In addition, the method may also comprise the step of encryption of data, and/or protection of data by a password or passcode, and/or locking data against deletion or modification. This step may be part of at least one or each of the steps described above, in particular of the steps using a transmitting unit 30.

The present invention is not limited to the embodiment as described herein above. All the features in the embodiments can be interchangeably combined as long as being covered by the appended claims. In particular, the invention is not limited to the shape, design, dimensions and composite materials of the container as long as they are suitable for the corresponding field of histology and thus also for storing the corresponding components 3, 4 and biological tissue samples even for a long time.

## Claims

1. A container (100, 300, 400) for collecting, transporting and storing a biological tissue sample of a human or animal, wherein the container (100, 300, 400) comprises a tag (10) with
a receiver (12.1) for receiving data relating to the biological tissue sample,
a non-volatile memory (11) for storing the data received by the receiver (12.1), and
a transmitter (12.2) for transmitting the data stored by the memory (11).

2. The container (100, 300, 400) according to claim 1, wherein the container (100, 300, 400) is filled with a component (3) being a fixative, wherein the container (100, 300, 400) is preferably filled with a further component (4) being a fluid such as a liquid, wherein the further component has a lower specific gravity than the fixative (3) and is immiscible with the fixative (3) such that the component (4) is stratified on top of the fixative (3).

3. The container (100, 300, 400) according to claim 1 or 2, wherein the data relating to the biological tissue sample includes a time stamp, wherein the time stamp is configured to relate to a fixation of the biological tissue sample contained in the container (100, 300, 400), in particular configured to indicate the beginning of fixation of the biological tissue sample contained in the container (100, 300, 400), wherein, preferably, the time stamp comprises a date, wherein the time stamp optionally comprises a fraction of the date, such as an hour and preferably a minute and more preferably a second, and/or wherein the time stamp optionally comprises a time zone and/or a format of time.

4. The container (100, 300, 400) according to any of the preceding claims, wherein the memory (11) is configured to store data including:
an identifier of the object from which the biological tissue sample is taken, such as a patient identifier and/or an organ identifier,
a dimension, a weight, and/or a parts count of the biological tissue sample, and/or
information regarding the fixative used in the container.

5. The container (100, 300, 400) according to any of the preceding claims, wherein the memory (11) comprises a first memory section for storing first data and a second memory section for storing second data, wherein each of the memory sections is configured to lock the respective data independently from the data of the respective other memory section, and/or
wherein the memory is configured to encrypt the data and/or to protect the data by a password or passcode.

6. The container (100, 300, 400) according to any of the preceding claims, wherein the tag comprises, or is, an RFID tag and/or an NFC tag.

7. The container (100, 300, 400) according to any of the preceding claims, further comprising a visual, machine-readable tag (20) such as a barcode, in particular a one- or two-dimensional barcode.

8. A system (500, 600, 700, 800, 900, 1000, 1100), comprising:
a container (100, 300, 400) according to any one of the preceding claims,
a transmitting unit (30) for sending data to be received by the receiver (12.1) of the container (100, 300, 400) and then stored in the memory (11) of the container (100, 300, 400), and
a reading unit (40) for reading data transmitted by the transmitter (12.2) of the container (100, 300, 400).

9. The system (500, 600, 700, 800, 900, 1000, 1100) according to claim 8, comprising a control unit (50) functionally connected to the transmitting unit (30) and the reading unit (40).

10. The system (500, 600, 700, 800, 900, 1000, 1100) according to claim 9, wherein the control unit (50) is configured to receive a signal indicating the presence of the container (100, 300, 400) and, upon receiving the signal, to activate the transmitting unit (30) in order to send, by the transmitting unit (30), data to the container (100, 300, 400), and/or to activate the reading unit (40) in order to read, by the reading unit (40), data transmitted by the container (100, 300, 400), wherein the reading unit (40) is preferably configured to generate the signal.

11. The system (500, 600, 700, 800, 900, 1000, 1100) according to claim 10, further comprising a user interface configured to generate the signal, wherein the user interface is preferably manually operable.

12. The system (500, 600, 700, 800, 900, 1000, 1100) according to any one of claims 8-11, wherein the data sent by the transmitting unit (30) and received by the receiver (12.1) of the container (100, 300, 400) includes a time stamp, in particular a time stamp configured to relate to a fixation of the biological tissue sample contained in the container (100, 300, 400), wherein the time stamp preferably comprises a date, wherein the time stamp more preferably comprises a fraction of the date, such as an hour and preferably a minute and more preferably a second, and/or wherein the time stamp optionally comprises a time zone and/or a format of time.

13. The system (600, 700, 800, 900, 1000, 1100) according to any one of claims 8-12, further comprising a visual reading unit (70) for reading a visual tag (20) comprised by the container (100, 300, 400), such as a barcode, in particular a one- or two-dimensional barcode, wherein the visual reading unit (70) is optionally configured to generate a signal upon reading the visual tag (20) and to transmit this signal to the control unit (50), for example as the signal indicating the presence of the container (100, 300, 400).

14. The system (600, 700, 800, 900, 1000, 1100) according to any one of claims 8-13, further comprising a base (60) on which the container (100, 300, 400) can be placed, wherein the base (60) comprises at least part of the transmitting unit (30) and/or at least part of the reading unit (40) and/or, if present, at least part of the control unit (50), wherein, preferably, a wall (80) extends from the base (60) so as to extend on a lateral side of the container (100, 300, 400) when the container (100, 300, 400) is placed on the base, wherein the wall (80) comprises at least part of the reading unit (30) and/or at least part of the transmitting unit (40) and/or, if present, at least part of the visual reading unit (70), wherein the wall (80) optionally comprises an opening (81), wherein the visual reading unit (70) is arranged to read a visual tag (20) via the opening (81).

15. A method, comprising the steps of:
a) providing a container (100, 300, 400) according to any one of claims 1-7,
b) transmitting, by a transmitting unit (30), data relating to a biological tissue sample,
c) receiving, by the receiver (12.1) of the container (100, 300, 400), the data, and
d) storing, by the non-volatile memory (11) of the container (100, 300, 400), the data received by the receiver (12.1).

16. The method according to claim 15, further comprising, preferably before step a), the step of placing the biological tissue sample in the container (100, 300, 400).
